# FASCICULE DE BREVET EUROPEEN

(11) **EP 1 345 880 B1**
(45) Date de publication et mention de la délivrance du brevet: **16.02.2005**
(21) Numéro de dépôt: 01995752.1
(22) Date de dépôt: 21.12.2001
(51) Int. Cl.: C07C 51/14, C07C 57/03

(54) **PROCEDE DE PREPARATION D'ACIDES CARBOXYLIQUES PAR CARBONYLATION AU PALLADIUM.**
VERFAHREN ZUR HERSTELLUNG VON KARBONSÄUREN DURCH KARBONYLIERUNG MIT PALLADIUM
METHOD FOR PREPARING CARBOXYLIC ACIDS BY PALLADIUM CARBONYLATION

(30) Priorité: 27.12.2000 FR 0017101
(43) Date de publication de la demande: 24.09.2003
(73) Titulaire: Rhodia Polyamide Intermediates, 69192 Saint-Fons (FR)
(72) Inventeur: SIMONATO, Jean-Pierre, F-38360 SASSENAGE (FR); METIVIER, Pascal, W4 4NU, LONDON (GB)
(74) Mandataire: Esson, Jean-Pierre
(86) Numéro de dépôt international: PCT/FR2001/004149
(87) Numéro de publication internationale: WO 2002/051783

(56) Documents cités:
- EP-A- 0 648 731
- FR-A- 2 529 885
- GB-A- 655 339

## Description

La présente invention concerne un procédé de préparation d'acides carboxyliques béta, gamma insaturés ou saturés.

Elle concerne plus particulièrement l'hydroxycarbonylation d'un composé organique comprenant une insaturation conjuguée, comme le butadiène, par action de monoxyde de carbone et d'eau en présence d'un catalyseur à base de palladium. Les acides carboxyliques ainsi obtenus sont de préférence, les acides penténoïques.

Une des voies possibles d'accès à l'acide adipique, qui est l'un des deux constituants de base des polyamides comme le polyamide 6.6, est la double carbonylation du butadiène ou de ses dérivés.

Bien qu'il puisse être imaginé de réaliser en une seule étape les deux hydroxycarbonylations menant du butadiène à l'acide adipique, il s'avère en pratique que les deux réactions doivent être conduites successivement, si l'on veut obtenir des sélectivités suffisamment élevées pour pouvoir envisager un procédé industriel économiquement viable.

Le brevet US-A-3 509 209 décrit l'hydroxycarbonylation de diverses oléfines, dont le butadiène, par le monoxyde de carbone et l'eau, en présence d'acide chlorhydrique ou bromhydrique et d'un catalyseur contenant du palladium, à une température de 15°C à 300°C et sous une pression de 1 à 1000 bars et de préférence de 10 à 200 bars.

Dans les conditions décrites, on observe que les rendements en acides penténoïques sont très faibles et qu'en réalité très souvent le produit obtenu est la valérolactone.

Le brevet FR-A-2 529 885 a proposé un procédé de préparation d'acides béta-gamma-insaturés tels que les acides penténoïques, par carbonylation d'un diène conjugué (butadiène plus particulièrement) en présence d'eau, d'un hydracide halogéné, d'un catalyseur à base de palladium et d'un sel d'onium quaternaire d'un élément choisi parmi l'azote, le phosphore et l'arsenic.

Ce procédé donne de bons résultats, mais il nécessite l'emploi d'une quantité relativement importante d'un sel d'onium quaternaire, composé onéreux et dont la présence est de nature à compliquer le traitement des mélanges en fin de réaction.

Le brevet européen 0648731 décrit également un procédé d'hydroxycarbonylation du butadiène et de ses dérivés en acides penténoïques en présence de chlorure de crotyle, à raison d'au moins deux moles par mole de palladium, le palladium se trouvant au moins en partie sous forme de complexe pi-crotyle. Ce procédé permet d'éviter l'utilisation de sel d'onium.

Ces différents procédés permettent la fabrication d'acides carboxyliques avec des rendements et sélectivités acceptables. Toutefois, la récupération et le recyclage du catalyseur n'a jamais été décrit dans ces documents. Comme le métal utilisé est un métal précieux, la récupération partielle de ce catalyseur ne permet pas un développement industriel de ces procédés.

Un des buts de la présente invention est de proposer un procédé d'hydroxycarbonylation comprenant une récupération totale ou sensiblement totale du catalyseur palladium. De plus, l'invention propose une récupération du palladium qui permet un recyclage de celui-ci dans une nouvelle étape d'hydroxycarbonylation.

A cet effet, l'invention propose un procédé de fabrication d'acides carboxyliques béta, gamma insaturés ou d'acides carboxyliques saturés par réaction d'un composé comprenant une insaturation éthylénique ou acétylénique conjuguée avec une autre insaturation ou un groupement donneur d'électrons porté par le carbone en position α de ladite insaturation, avec du monoxyde de carbone et de l'eau. Cette réaction est réalisée en présence d'un catalyseur à base de palladium soluble dans le milieu.

Le procédé de l'invention se caractérise en ce que, le milieu réactionnel, en fin de réaction d'hydroxycarbonylation, est traité, dans une première étape, pour extraire le monoxyde de carbone présent dans le milieu par évaporation ou entrainement du CO, puis dans une seconde étape, pou réduire le palladium présent dans le milieu à du palladium à l'état d'oxydation zéro, par traitement avec de l'hydrogène. Le palladium ainsi réduit précipite et peut être séparé du milieu réactionnel par tout moyen usuel de séparation solide/liquide.

Selon une caractéristiques de l'invention, la concentration en CO du milieu avant traitement par l'hydrogène est avantageusement inférieure à 600 ml de CO par litre de solution, de préférence inférieure à 100ml de CO par litre de solution. La concentration en CO est également de préférence supérieure à 0,001 ml de CO par litre de solution.

Par composés comprenant une insaturation, il faut comprendre les composés de formules générales :

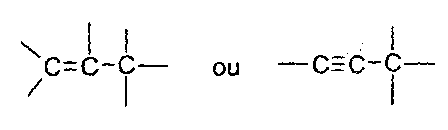

Dans lesquelles le carbone en position alpha par rapport à l'insaturation éthylénique ou acétylénique porte également une insaturation éthylénique ou un radical donneur d'électrons comme, par exemple, des atomes d'halogènes, des groupements amino substitués ou non, des groupements alcoxy, hydroxy, oxo, époxy, carbonyle, mercapto-alcoyle, ester et/ou cyano.

A titre d'exemple de tels composés insaturés, on peut citer les dioléfines, les alcools allyliques, éthers allyliques, esters allyliques et halogénures allyliques.

Le composé préféré de l'invention est le butadiène permettant de fabriquer des acides penténoïques par simple hydroxycarbonylation, ou l'acide adipique par double hydroxycarbonylation.

Le catalyseur de l'invention est un composé du palladium soluble dans le milieu réactionnel. Les composés pour la mise en oeuvre de l'invention sont ceux décrits dans les documents cités ci-dessus pour illustrer l'état de la technique.

On peut citer, par exemple, les composés de palladium contenant un ou plusieurs groupements anioniques tels que, par exemple, ceux dérivés des acides chlorhydrique, iodhydrique, fluorhydrique ou bromhydrique, l'acide sulfurique, l'acide nitrique, l'acide carbonique.

Sont également convenables, les groupements anioniques dérivés des acides sulfoniques, des thioalcools, des acides carboxyliques comme l'acide acétique, l'acide propionique, l'acide pivalique ou analogues.

On peut également utiliser des complexes formés à partir de composés comprenant des atomes de phosphore ou d'azote.

Le palladium peut également être présent sous forme de complexes organiques qui peuvent être formés avant l'introduction dans le milieu réactionnel ou directement dans ledit milieu.

Des exemples de complexes utilisables dans le présent procédé sont :
bis(chlorure de pi-allylpalladium), bis(bromure de pi-allylpalladium), acétylacétonato(allyl)palladium, bis(chlorure de pi-isobutènylpalladium), bis (chlorure de pi-cyclohexénylpalladium) et d'autres complexes de pi-allyle comme le bis(chlorure de pi-4-chlorocrotylpaliadium) et le bis(chlorure de pi-2-méthyl-4-chlorocrotylpalladium), les chlorures de pi-allylcarbonylpalladium et chlorure de pi-isobutényl-carbonyl-palladium, respectivement par exemple (C₄H₇Pd₂Cl₂CO)₂) et, de plus, le chlorure d'éthylènepalladium.

Il est également possible d'utiliser des complexes organiques du palladium tels que l'acétylacétonate de palladium, le bis(bi benzylidène acétone)palladium, le dimère de chlorure crotyle de palladium, les complexes de phosphines aromatiques ou aliphatiques avec du palladium comme la tétrakistriphénylphosphine de palladium, le dichlorure de palladium bistriphénylphosphine.

Selon un mode de réalisation préféré de l'invention, le catalyseur au palladium est formé in-situ par addition de chlorure de crotyle, à raison d'au moins deux moles par mole de palladium. Le palladium se trouve ainsi au moins partiellement sous forme de complexe pi-crotyle, comme décrit dans le brevet européen n° 0648731.

Selon l'invention, le procédé d'hydroxycarbonylation de composés organiques insaturés décrits ci-dessus et qui seront exemplifiés pour plus de clarté par référence au butadiène, par le monoxyde de carbone et l'eau est mis en oeuvre avantageusement à une pression en monoxyde de carbone supérieure à la pression atmosphérique.

Avantageusement, l'eau présente dans le milieu réactionnel représente une quantité inférieure ou égale à 20 % en poids par rapport au poids du mélange réactionnel.

Par dérivé du butadiène; on entend dans le présent texte notamment les buténols allyliques comme le 3-butène-2-ol, le 2-butène-1-ol et leurs mélanges, les composés d'addition du chlorure d'hydrogène sur le butadiène (chlorobutènes) dont le principal est le chlorure de crotyle.

Dans le présent procédé, on peut mettre en oeuvre le butadiène, un ou plusieurs de ses dérivés ou les mélanges du butadiène avec un ou plusieurs de ses dérivés. Le butadiène ou les mélanges contenant une partie majoritaire de butadiène représentent cependant les substrats préférés.

Le catalyseur complexe pi-crotyl-palladium peut être introduit dans le milieu réactionnel ou être formé in situ à partir des halogénures de Pd, plus particulièrement le chlorure, des carboxylates de Pd, notamment l'acétate ou encore à partir de palladium métallique finement divisé.

La quantité de catalyseur pi-crotyl-palladium utilisée dans le procédé peut varier dans de larges limites. Généralement on met en oeuvre de 10⁻⁵ mole à 0,2 mole de Pd par mole de butadiène ou de dérivé du butadiène engagé dans la réaction et de préférence de 10⁻⁴ mole à 0,1 mole par mole.

Outre le catalyseur pi-crotyl-palladium peut se trouver également dans le milieu réactionnel du palladium sous une autre forme moins active (par exemple du Pd métallique ou du chlorure de Pd) en quantité variable. Dans un procédé industriel, il est cependant préférable que tout ou pratiquement tout le palladium soit sous une forme active et soluble dans le milieu, comme le pi-crotyl-palladium, éventuellement avec du chlorure de palladium.

Le complexe pi-crotyl-palladium peut être préparé par exemple en faisant réagir un sel de palladium, comme le chlorure de palladium, avec du chlorure de crotyle, dans un solvant pouvant être constitué par un mélange eau/méthanol. Le mélange est agité, généralement à température ambiante, avantageusement sous léger courant de monoxyde de carbone. Le complexe pi-crotyl-palladium précipite. Après une éventuelle phase de dégazage, le mélange est versé dans de l'eau, puis est extrait à l'aide d'un solvant organique adapté, tel que par exemple du chloroforme. Le complexe est ensuite isolé de la solution organique par évaporation du solvant.

Le promoteur chlorure de crotyle peut être introduit dans le mélange réactionnel ou être formé in situ, à partir de butadiène et/ou de 2-butène-1-ol et d'acide chlorhydrique.

Il représente, en mole par mole, de préférence de 5 à 10 fois la quantité de palladium, bien qu'il puisse être présent en plus grandes proportions, puisqu'il peut constituer tout ou partie du substrat à hydroxycarbonyler.

Globalement il est préférable d'avoir dans le milieu réactionnel un rapport molaire CI/Pd inférieur ou égal à 100 et de préférence inférieur ou égal à 20, car des rapports élevés ont une influence néfaste sur la cinétique de la réaction.

Comme indiqué précédemment, la concentration en eau dans le mélange réactionnel est, avantageusement, maintenue à une valeur égale ou inférieure à 20 % en poids par rapport au poids dudit mélange. En effet la concentration en eau a un effet sur la cinétique de la réaction. De préférence cette concentration en eau sera maintenue à une valeur égale ou inférieure à 8 % en poids et encore plus préférablement à une valeur égale ou inférieure à 5 %.

L'eau étant un réactif indispensable à la réaction d'hydroxycarbonylation, une variante intéressante du procédé de l'invention consiste à injecter cette eau au fur et à mesure de l'avancement de la réaction, ce qui permet de maintenir sa concentration dans le mélange réactionnel à une valeur très faible, tout en permettant à la réaction de s'effectuer.

Bien que la présence d'un tiers-solvant ne soit pas exclue, la réaction est généralement conduite sans solvant autre que les réactifs eux-mêmes ou les produits de la réaction. Il peut également être favorable d'introduire dés le début de la réaction d'hydroxycarbonylation un acide penténoïque, et plus particulièrement l'acide pentène-3-oïque, afin de minimiser les réactions secondaires.

Dans le cadre d'une mise en oeuvre industrielle du procédé, des recyclages du butadiène n'ayant pas réagi, peuvent conduire à l'introduction dans le milieu réactionnel de quantités plus ou moins importantes d'autres composés, notamment de sous-produits formés lors de la réaction d'hydroxycarbonylation. Ainsi, on peut avoir dans le mélange réactionnel par exemple des butènes, de la gamma-valérolactone, de l'acide valérique, de l'acide adipique, de l'acide méthyl-2 glutarique, de l'acide éthyl-2 succinique, de l'acide méthyl-2-butanoïque, des acides méthyl-2-buténoïques. Compte tenu des impératifs d'une possible mise en oeuvre en continu du procédé, les quantités présentes de ces composés peuvent atteindre jusqu'à 90 % en poids du mélange réactionnel engagé dans la réaction d'hydroxycarbonytation.

La concentration en butadiène est un paramètre important de la réaction, notamment pour ce qui concerne la stabilité du catalyseur au palladium, c'est-à-dire essentiellement son maintien en solution dans le mélange réactionnel. Il a ainsi été observé qu'il n'est pas favorable d'avoir moins de 0,2 % en poids de butadiène par rapport au poids total du mélange réactionnel. De préférence, lorsque l'on opère en discontinu, la conversion du butadiène ou de ses dérivés sera limitée de manière à ce que le mélange réactionnel renferme au moins 0,5 % en poids dudit butadiène ou de ses dérivés.

La concentration en butadiène sera également de préférence maintenue à une valeur égale ou inférieure à 50 % en poids par poids du mélange réactionnel et encore plus préférentiellement à une valeur égale ou inférieure à 30 % , lorsque l'on opère en procédé discontinu et à une valeur égale ou inférieure à 10 % lorsque l'on opère en procédé continu.

Le mode de réalisation du procédé d'hydroxycarbonylation décrit ci-dessus présente l'avantage par rapport à d'autres modes de réalisation, de maintenir le palladium en solution dans le milieu réactionnel jusqu'à la fin de la réaction permettant de maintenir une cinétique élevée.

La réaction d'hydroxycarbonylation peut être conduite à une température généralement située entre 60°C et 230°C et de préférence entre 90°C et 200°C et sous une pression en température de 50 à 500 bars et de préférence de 100 à 300 bar.

La pression.partielle de monoxyde de carbone, mesurée à 25°C, est de 25 bar à 440 bar et de préférence de 55 bar à 240 bar.

Comme cela a été indiqué, le procédé de l'invention peut être mis en oeuvre de manière continue ou discontinue. Selon la mise en oeuvre choisie, il y aura donc lieu d'adapter les différentes conditions opératoires définies précédemment.

Le milieu réactionnel obtenu en fin de réaction d'hydroxycarbonylation contient le palladium sous forme dissoute.

L'invention propose un traitement de ce milieu réactionnel permettant de récupérer le palladium sous forme insoluble, avec un taux de récupération voisin de 100%.

Ce traitement consiste dans une première étape à extraire le monoxyde de carbone dissous dans le milieu réactionnel. Cette extraction peut être obtenue par chauffage du milieu avec une atmosphère ne contenant pas de CO. Plus avantageusement, cette extraction est réalisée par mise sous atmosphère inerte ou d'hydrogène du milieu réactionnel avec, éventuellement, un barbotage de ce gaz inerte ou de l'hydrogène, dans le milieu réactionnel. Par gaz inerte, on entend l'azote ou les gaz rares.

Cette étape peut être réalisée à une température comprise entre 20°C (température ambiante) et 150°C.

Cette opération est réalisée pour extraire pratiquement la totalité du monoxyde de carbone extractible du milieu réactionnel. Ainsi, la concentration en monoxyde de carbone dans le milieu réactionnel pourra être avantageusement inférieure à 600 ml/l, de préférence inférieure à 100 ml/l de solution. Avantageusement, la concentration en CO dans le milieu réactionnel est comprise entre 0,001ml/l et 600 ml/l.

Après extraction du monoxyde de carbone le procédé de l'invention comprend une étape de réduction du palladium dissous, généralement à l'état d'oxydation 2+, en du palladium à l'état d'oxydation zéro (Pd⁰). Ce palladium est séparé du milieu réactionnel par les techniques habituelles de séparation liquide/solide telles que, la filtration, la décantation, la centrifugation, la distillation ou évaporation du milieu liquide.

Selon un mode de réalisation préféré de l'invention, un composé insoluble dans le milieu réactionnel est présent dans ledit milieu avant la précipitation du Pd⁰.

Ce composé insoluble peut être ajouté dans le milieu réactionnel à tout moment, par exemple au début du procédé d'hydroxycarbonylation, à la fin de celui-ci ou juste avant l'étape de réduction du palladium.

Avantageusement, ce composé insoluble est ajouté avant le début de la réaction d'hydroxycarbonylation.

Selon l'invention, le palladium à l'état d'oxydation zéro précipite et se dépose sur ou dans la phase hétérogène constituée par ce composé insoluble, facilitant ainsi son extraction et sa séparation du milieu réactionnel.

De manière générale, ce composé insoluble ne devra pas avoir d'effet sur la réaction d'hydroxycarbonylation, notamment ne pas affecter l'effet catalytique du palladium ou la sélectivité de la réaction.

Comme composés insolubles convenables pour l'invention, on peut citer les composés minéraux avantageusement poreux ou présentant une grande surface spécifique. Dans cette famille de composés, on peut citer à titre d'exemple, les charbons actifs, l'alumine, la silice, la zircone, l'oxyde de cérium, ou plus généralement les oxydes de terres rares.

Comme autres composés insolubles convenables pour l'invention, on peut citer les mousses polymériques telles que les mousses de polystyrène ou les huiles silicones.

La quantité ajoutée de composés insolubles n'est pas critique et peut varier dans de grandes proportions.

Selon une caractéristique de l'invention, l'étape de réduction du palladium à l'état d'oxydation zéro est réalisée par mise sous atmosphère d'hydrogène du milieu réactionnel.

Toutefois, il est également possible, sans sortir du cadre de l'invention, d'ajouter un composé réducteur dans le milieu tels que les hydrures métalliques ou les borohydrures comme NaBH₄. par exemple.

Cette étape de réduction peut être conduite entre la température ambiante (20°C) et 150°C et une pression en hydrogène comprise entre 1 bar et 100 bar.

Toutefois, dans un mode de réalisation permettant de réduire le palladium à l'état d'oxydation zéro sans affecter la ou les insaturations présentes dans le composé hydrocarbonylé, la température de l'étape de réduction est avantageusement comprise entre 20°C (température ambiante) et 80°C.

Dans l'autre mode de réalisation, avec une température comprise entre 80°C et 150°C, les insaturations de l'acide carboxylique obtenu par hydrocarbonylation peuvent être hydrogénées pour obtenir un acide carboxylique saturé. Le procédé de l'invention permet ainsi, à partir du butadiène, de produire de l'acide pentanoique.

Le procédé de l'invention permet de récupérer la totalité du palladium présent dans le milieu réactionnel.

En outre, le composé insoluble contenant le palladium peut être traité par des acides pour remettre en solution le palladium et permettre son recyclage dans une nouvelle étape d'hydroxycarbonylation.

Le procédé de l'invention permet donc une mise en oeuvre des réactions d'hydroxycarbonylation de composés insaturés avec catalyse au palladium sans perte de catalyseur. Un tel procédé est donc économiquement exploitable.

L'invention sera mieux illustrée par les exemples donnés ci-dessous uniquement à titre illustratif et sans effet limitatif.

### Exemple 1

Dans une ampoule de verre, on charge :

| | |
|---|---|
| Acide 3-pentenoïque (90% de pureté) | 10,0 g |
| Butadiène | 2,7 g (50 mmol) |
| Chlorobutène | 0,3 g (0,35 mmol) |
| Eau | 0,9 g (50 mmol) |
| Palladium sur charbon actif (3%) | 0,71 g |

Ce catalyseur Pd/C est commercialisé par la société ENGELHARD sous la dénomination commerciale ESCAT® 162 5207

L'ampoule de verre est introduite dans un autoclave de 125 ml. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est élevée à 200 bar avec communication avec une réserve de CO pour maintenir une pression constante. La consommation de CO est estimée par rapport à la différence de pression mesurée dans la réserve.

Après 40 minutes de réaction correspondant à un taux de transformation (TT) du butadiène de 63%, l'autoclave est refroidi dans un bain d'eau à 20°C puis dégazé. Trois purges à l'hydrogène sont réalisées sous une pression de 20 bar d'hydrogène. La teneur en CO dans le milieu est de 0,6 ml de CO par litre de solution.

Une pression de 20 bar d'hydrogène est appliquée et le mélange est chauffé à 80°C sous agitation pendant une heure (phase de réduction du palladium). Après refroidissement et dégazage, le milieu réactionnel est filtré. Le charbon recueilli est séché avant analyse.

La masse réactionnelle recueillie après filtration est analysée par chromatographie liquide à haute performance (HPLC) et chromatographie en phase gazeuse (CPG).

Les résultats suivants ont été obtenus :
RT (acide 3-pentènoïque) = 92%
RT (diacides) = 4,6 %
RT(acide 2-méthyl-3-butènoïque) = 2,9%
RT(acide pentanoïque) = 0,07%

Le dosage du palladium sur le charbon recueilli et dans la masse réactionnelle après filtration est réalisé par ICP-optique ou ICP-masse. Les résultats montrent que 98 % du palladium engagé dans la réaction est récupéré sur le charbon et 1 % se trouve sous forme dissoute dans le milieu réactionnel.

Par taux de transformation (TT) du butadiène il faut comprendre le rapport exprimé en % du nombre de moles de butadiène ayant disparu au nombre de moles de butadiène engagées.

Par RT il faut comprendre la sélectivité en produit X indiqué correspondant au rapport exprimé en % entre le nombre de moles de produit X formées et le nombre de moles théorique de produit X calculé à partir du nombre de moles de butadiène transformées.

### Exemple 2

Dans une ampoule en verre, on charge

| | |
|---|---|
| acide 3-pentènoïque (90% de pureté) | 10,0 g |
| Butadiène | 2,6 g |
| Chlorobutène | 0,15 g |
| Eau | 0,9 g |
| Acétate de palladium | 0,10 g |
| Charbon CECA L3S | 1,1 g |

Le charbon est commercialisé par la société CECA

L'ampoule de verre est introduite dans un autoclave de 125 mL. Celui-ci est immédiatement pressurisé à 100 bar de CO à température ambiante. L'autoclave est placé dans un four et le mélange est chauffé à 140°C. sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

Après 19 minutes de réaction correspondant à un TT du butadiène égal à 65%, l'autoclave est refroidi dans un bain d'eau à 20°C puis dégazé. Deux purges à l'hydrogène sont réalisées sous 20 bar d'hydrogène. La solution obtenue contient 40 ml de CO par litre de solution.

Une pression de 20 bar d'hydrogène est appliquée et le mélange est chauffé à 40°C sous agitation pendant quinze minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 83%
RT (diacides) = 11 %
RT(acide 2-méthyl-3-butènoïque) = 3,6 %
RT(acide pentanoïque) = 1,8 %

Le dosage du palladium sur le charbon recueilli et dans la masse réactionnelle après filtration est réalisé par ICP-optique ou ICP-masse. Les résultats montrent que 1 % du palladium engagé dans la réaction se trouve sous forme dissoute dans le milieu réactionnel, le reste étant récupéré sur le charbon.

### Exemple 3

Dans une ampoule en verre, on charge

| | |
|---|---|
| acide 3-pentènoïque (90% de pureté) | 10,0 g |
| Butadiène | 3,2 g |
| Chlorobutène | 0,32 g |
| Eau | 0,9 g |
| Acétate de palladium | 0,10 g |
| Charbon CECA L3S | 1,0 g |

Le charbon est commercialisé par la société CECA

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

Après 23 minutes de réaction correspondant à un TT du butadiène égal à 70%, l'autoclave est refroidi dans un bain d'eau à 20°C. L'autoclave est détendu et mis sous une pression de 60 bar avec un mélange H₂/CO à teneur pondérale 95/5. L'autoclave est chauffé à 40° durant 45 minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 87%
RT (diacides) = 10 %
RT (acide pentanoïque) = 0,25%
RT(acide 2-méthyl-3-butènoïque) = 3,2%

Le dosage du palladium sur le charbon recueilli et dans la masse réactionnelle après filtration est réalisé par ICP-optique ou ICP-masse. Les résultats montrent qu'uniquement 34 % du palladium engagé dans la réaction est récupéré sur le charbon et 62 % se trouve sous forme dissoute dans le milieu réactionnel.

### Exemple 4

Dans une ampoule en verre, on charge

| | |
|---|---|
| acide 3-pentènoïque (90% de pureté) | 10,2 g |
| Butadiène | 2,7 g |
| Chlorobutène | 0,20 g |
| Eau | 0,9 g |
| Palladium sur charbon actif (3%) | 1,0 g |

Ce catalyseur Pd/C est commercialisé par la société ENGELHARD sous la dénomination commerciale ESCAT® 162 5207

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

Après 24 minutes de réaction correspondant à un TT du butadiène égal à 51%, l'autoclave est refroidi dans un bain d'eau à 20°C puis dégazé lentement. Aucune purge n'est réalisée, la concentration en CO étant de 1000ml de CO par litre de solution. Une pression de 20 bar d'hydrogène est alors appliquée et le mélange est chauffé à 40°C sous agitation pendant quinze minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 87%
RT (diacides) = 10 %
RT(acide pentanoïque) = 0,3 %

Le dosage du palladium sur le charbon recueilli et dans la masse réactionnelle après filtration est réalisé par ICP-optique ou ICP-masse. Les résultats montrent que 88 % du palladium engagé dans la réaction se trouve sous forme dissoute dans le milieu réactionnel.

### Exemple 5

Dans une ampoule en verre, on charge

| | |
|---|---|
| acide 3-pentènoïque (90% de pureté) | 10,0 g |
| Butadiène | 2,7 g |
| Chlorobutène | 0,30 g |
| Eau | 0,9 g |
| Acétate de palladium | 0,11 g |
| Charbon CECA L3S | 1,0 g |

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

Après 14 minutes de réaction correspondant à un TT du butadiène égal à 64%, l'autoclave est refroidi dans un bain d'eau à 20°C puis dégazé lentement. Aucune purge n'est réalisée. Une pression de 20 bar d'hydrogène est alors appliquée et le mélange est chauffé à 80°C sous agitation pendant quinze minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 80%
RT (diacides) = 15 %
RT(acide pentanoïque) = 0,2 %
RT(acide 2-méthyl-3-butènoïque) = 3,8%

Le dosage du palladium sur le charbon recueilli et dans la masse réactionnelle après filtration est réalisé par ICP-optique ou ICP-masse. Les résultats montrent que 80 % du palladium engagé dans la réaction se trouve sous forme dissoute dans le milieu réactionnel.

### Exemple 6

Dans une ampoule en verre, on charge

| | |
|---|---|
| acide 3-pentènoïque (90% de pureté) | 10,0 g |
| Butadiène | 2,6 g |
| Chlorobutène | 0,30 g |
| Eau | 0,91 g |
| Acétate de palladium | 0,10 g |
| Charbon CECA L3S | 1,0 g |

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

En fin de réaction l'autoclave est refroidi dans un bain d'eau à 20°C puis dégazé lentement. Une purge est réalisée avec 40 bar d'hydrogène, la concentration en CO tombe à 40 ml de CO par litre de solution. Une pression de 20 bar d'hydrogène est alors appliquée et le mélange est chauffé à 40°C sous agitation pendant quinze minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 80%
RT (diacides) = 12 %
RT(acide pentanoïque) = 2,1 %
RT(acide 2-méthyl-3-butènoïque) = 3,9%

Le dosage du palladium sur le charbon recueilli et dans la masse réactionnelle après filtration est réalisé par ICP-optique ou ICP-masse. Les résultats montrent que 1 % du palladium engagé dans la réaction se trouve sous forme dissoute dans le milieu réactionnel.

### Exemple 7

Dans une ampoule en verre, on charge

| | |
|---|---|
| acide 3-pentènoïque (90% de pureté) | 10,0 g |
| Butadiène | 2,8 g |
| Chlorobutène | 0,31 g |
| Eau | 0,9 g |
| Acétate de palladium | 0,10 g |
| Charbon CECA L3S | 1,0 g |

Le charbon est commercialisé par la société CECA

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

En fin de réaction, l'autoclave est refroidi dans un bain d'eau à 20°C. L'autoclave est détendu puis trois purges à l'hydrogène sont réalisées sous 20 bar d'hydrogène (la concentration en CO tombant à 0,6 ml par litre de solution) puis une pression de 10 bar d'hydrogène est appliquée et le mélange est chauffé à 80°C sous agitation pendant cinq minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide 3-pentènoïque) = 81%
RT(acide 4-pentènoïque) = 3,7 %
RT (diacides) = 8,5 %
RT(acide 2-méthyl-3-butènoïque) = 3,3%
RT(acide pentanoïque) = 4,4 %

Le dosage du palladium sur le charbon recueilli et dans la masse réactionnelle après filtration est réalisé par ICP-optique ou ICP-masse. Les résultats montrent que 97 % du palladium engagé dans la réaction est récupéré sur le charbon et 1 % se trouve sous forme dissoute dans le milieu réactionnel.

### Exemple 8

Dans une ampoule en verre, on charge

| | |
|---|---|
| acide 3-pentènoïque (90% de pureté) | 10,0 g |
| Butadiène | 2,7 g |
| Chlorobutène | 0,30 g |
| Eau | 0,95 g |
| Acétate de palladium | 0,10 g |
| Alumine Degussa type C | 1,0 g |

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

En fin d'essai l'autoclave est refroidi dans un bain d'eau à 20°C puis dégazé. Trois purges à l'hydrogène sont réalisées sous 20 bar d'hydrogène (la concentration en CO tombant à 0,6 ml par litre de solution) puis une pression de 20 bar d'hydrogène est appliquée et le mélange est chauffé à 80°C sous agitation pendant quarante cinq minutes. Après refroidissement et dégazage, le milieu réactionnel est filtré. L'alumine récupérée est séchée avant analyse.

Le dosage du palladium sur l'alumine recueillie et dans la masse réactionnelle après filtration est réalisé par ICP-optique ou ICP-masse. Les résultats montrent que 97 % du palladium engagé dans la réaction est récupéré sur le charbon et moins de 0,05 % se trouve sous forme dissoute dans le milieu réactionnel.

### Exemple 9

### 1) Préparation du complexe chlorure de pi-crotyl-Pd.

Dans un ballon en verre de 150 cm³ , on charge successivement 5,04 g de PdCl₂, 3,37 g de NaCl, 50 cm³ de méthanol, 15 cm³ d'eau, 8,03 g de chlorure de crotyle et à nouveau 20 cm³ de méthanol.

On agite le mélange hétérogène qui devient progressivement marron foncé et trouble. On traite ensuite la solution sous agitation par un léger courant de monoxyde de carbone (bulle à bulle) pendant une heure. Le mélange s'éclaircit et un précipité jaune apparaît. L'agitation et le courant de CO sont arrêtés, la solution est abandonnée pendant une heure au repos, puis elle est versée dans 300 cm³ d'eau, extraite par 5 fois 50 cm³ de chloroforme. La phase organique résultante jaune paille est lavée par 2 fois 100 cm³ d'eau, séchée sur sulfate disodique pendant une nuit, puis le solvant est évaporé. On récupère ainsi 3,35 g d'un solide jaune pâle ayant une pureté supérieure à 94 % (dosage par Résonance Magnétique Nucléaire : RMN).

### 2) Hydroxycarbonylation du chlorobutène :

On charge dans une ampoule de verre :

| | |
|---|---|
| Chlorobutène | 5,0 g |
| Palladium chlorocrotyle | 10,3 mg |

L'ampoule de verre est introduite dans un autoclave de 125 ml. Celui-ci est immédiatement pressurisé à 100 bar de CO à température ambiante. L'autoclave est placé dans un four et le mélange est chauffé à 130°C, sous agitation par secousses. Lorsque la température de consigne est atteinte, la pression de CO est amenée à 200 bar et l'autoclave est mis en communication avec une réserve de CO pour maintenir une pression constante.

Après 60 minutes, l'autoclave est refroidi dans un bain d'eau à 20°C puis dégazé. La masse réactionnelle homogène est analysée par chromatographie liquide haute performance (HPLC) et chromatographie en phase gazeuse (CPG).

Les résultats suivants ont été obtenus :
RR (P3) = 12,7%
RR (P2) = 4,4%

Le reste est très majoritairement du chlorobutène.

2,09 g de la solution ainsi obtenue sont placés dans une ampoule de verre et 95 mg de charbon CECA L3S sont ajoutés. Deux purges sous 15 bar d'hydrogène sont réalisées et le mélange est mis sous agitation par secousses sous 20 bar d'H₂ et chauffé à 80°C. Une fois la température atteinte, une pression de 40 bar d'hydrogène est appliquée pendant 60 minutes. L'autoclave est dégazé et le milieu réactionnel filtré.

Le dosage du palladium sur le charbon recueilli et dans la masse réactionnelle après filtration est réalisé par ICP-optique ou ICP-masse. Les résultats montrent que 100 % du palladium engagé dans la réaction est récupéré sur le charbon.

### Exemple 10

Dans une ampoule en verre, on charge

| | |
|---|---|
| acide pentanoïque | 10,0 g |
| Butadiène | 2,6 g |
| Chlorobutène | 0,30 g |
| Eau | 0,92 g |
| Acétate de palladium | 0,11 g |
| Charbon CECA L3S | 1,0 g |

L'ampoule de verre est introduite dans un autoclave de 125 mL. Celui-ci est immédiatement pressurisé à 100 bar de CO à température ambiante. L'autoclave est placé dans un four et le mélange est chauffé à 140°C, sous agitation par secousses. Lorsque la température de consigne est atteinte, la pression de CO est amenée à 200 bar et l'autoclave mis en communication avec une réserve de CO pour maintenir une pression constante. La consommation de CO est estimée par rapport à la différence de pression mesurée dans la réserve.

En fin de réaction l'autoclave est refroidi dans un bain d'eau à 20°C puis dégazé lentement. Cinq purges sont effectuées sous 60 bar d'hydrogène (la concentration en CO tombant à 0,05 ml par litre de solution). Le mélange est alors chauffé à 140°C sous 70 bar d'hydrogène sous agitation pendant trente cinq minutes. Après refroidissement et dégazage, le milieu réactionnel est filtré. Le charbon récupéré est séché avant analyse. La masse réactionnelle est analysée par chromatographie liquide à haute performance (HPLC) et chromatographie en phase gazeuse (CPG).

Les résultats suivants ont été obtenus :
RT (acide pentanoïque) = 93%
RT (diacides) = 3,0 %
RT(acide 2-méthyl-3-butanoïque) = 3,9%

Le dosage du palladium sur le charbon recueilli et dans la masse réactionnelle après filtration est réalisé par ICP-optique ou ICP-masse. Les résultats montrent que presque 100% du palladium engagé dans la réaction est récupéré sur le charbon, 0,025 % se trouvant sous forme dissoute dans le milieu réactionnel.

## Revendications

1. Procédé de préparation d'acide carboxyliques insaturés ou saturés à partir de composés comprenant une insaturation éthylénique ou acétylénique conjuguée avec une autre insaturation ou un groupement donneur d'électrons, par réaction de monoxyde de carbone et d'eau en présence d'un catalyseur à base de palladium, **caractérisé en ce qu'**il consiste à traiter le milieu réactionnel après la fin de la réaction d'hydroxycarbonylation par extraction du monoxyde de carbone dudit milieu réactionnel par traitement avec un gaz pour obtenir une concentration en CO inférieure à 600 ml de CO par litre de solution, puis traitement du milieu réactionnel par de l'hydrogène pour réduire le palladium à l'état d'oxydation zéro, et à séparer le palladium précipité du milieu réactionnel.

2. Procédé selon la revendication 1, **caractérisé en ce que** la concentration en monoxyde de carbone dans le milieu réactionnel avant l'étape de traitement par l'hydrogène est inférieure à 10 mmol/l.

3. Procédé selon la revendication 2, **caractérisé en ce que** la concentration en monoxyde de carbone dans le milieu réactionnel avant l'étape de traitement par l'hydrogène est comprise entre 0,001 ml/l et 600 ml/l.

4. Procédé selon l'une des revendications 1 à 3, **caractérisé en ce que** le gaz d'entraînement du monoxyde de carbone est choisi dans le groupe comprenant l'azote, les gaz rares ou l'hydrogène.

5. Procédé selon l'une des revendications 1 à 4, **caractérisé en ce que** l'étape de réduction du palladium à l'état d'oxydation zéro est mise en oeuvre sous une pression d'hydrogène comprise entre 1 bar et 100 bar.

6. Procédé selon l'une des revendications 1 à 5, **caractérisé en ce que** l'étape de réduction du palladium à l'état d'oxydation zéro est mise en oeuvre à une température comprise entre 20°C et 150°C.

7. Procédé selon l'une des revendications précédentes, **caractérisé en ce qu'**un composé non soluble dans le milieu réactionnel est ajouté dans ledit milieu, puis séparé dudit milieu après l'étape de réduction du palladium à l'état d'oxydation zéro.

8. Procédé selon la revendication 7 **caractérisé en ce que** le composé insoluble dans le milieu réactionnel est choisi dans le groupe comprenant les charbons actifs, l'alumine, la silice, la zircone, l'oxyde de cérium, les mousses polystyréniques, les huiles silicones.

9. Procédé selon la revendication 7 ou 8, **caractérisé en ce que** le composé insoluble dans le milieu réactionnel est ajouté en début de réaction.

10. Procédé selon la revendication 7 ou 8, **caractérisé en ce que** le composé insoluble dans le milieu réactionnel est ajouté avant l'étape de réduction du palladium.

11. Procédé selon l'une des revendications précédentes, **caractérisé en ce que** les composés comprenant une insaturation éthylénique conjuguée sont choisis dans le groupe comprenant les dioléfines, les alcools allyliques, éthers allyliques, esters allyliques et halogénures allyliques.

12. Procédé selon la revendication 11, **caractérisé en ce que** la dioléfine est le butadiène.

13. Procédé selon l'une des revendications 7 à 12, **caractérisé en ce que** le palladium déposé sur le composé insoluble est récupéré par traitement dudit composé insoluble après séparation du milieu réactionnel.

14. Procédé selon la revendication 13, **caractérisé en ce que** le traitement du composé insoluble comprenant le palladium réduit est une dissolution du palladium par attaque par un acide fort.

## Claims

1. Process for the preparation of unsaturated or saturated carboxylic acids from compounds comprising an ethylenic or acetylenic unsaturation-conjugated with another unsaturation or an electron-donating group by reaction of carbon monoxide and water in the presence of a palladium-based catalyst, **characterized in that** it consists in treating the reaction medium, after the end of the hydroxycarbonylation reaction, by extraction of the carbon monoxide from the said reaction medium by treatment with a gas, in order to obtain a CO concentration of less than 600 ml of CO per litre of solution, in then treating the reaction medium with hydrogen to reduce the palladium to the zero oxidation state, and in separating the precipitated palladium from the reaction medium.

2. Process according to claim 1, **characterized in that** the concentration of carbon monoxide in the reaction medium before the stage of treatment with hydrogen is less than 10 mmol/l.

3. Process according to claim 2, **characterized in that** the concentration of carbon monoxide in the reaction medium before the stage of treatment with hydrogen is between 0.001 ml/l and 600 ml/l.

4. Process according to one of claims 1 to 3, **characterized in that** the gas for entrainment of the carbon monoxide is chosen from the group consisting of nitrogen, the rare gases or hydrogen.

5. Process according to one of claims 1 to 4, **characterized in that** the stage of reduction of the palladium to the zero oxidation state is carried out under a hydrogen pressure of between 1 bar and 100 bar.

6. Process according to one of claims 1 to 5, **characterized in that** the stage of reduction of the palladium to the zero oxidation state is carried out at a temperature of between 20°C and 150°C.

7. Process according to one of the preceding claims, **characterized in that** a compound which is insoluble in the reaction medium is added to the said medium and then separated from the said medium after the stage of reduction of the palladium to the zero oxidation state.

8. Process according to claim 7, **characterized in that** the compound which is insoluble in the reaction medium is chosen from the group consisting of active charcoals, alumina, silica, zirconia, cerium oxide, polystyrene foams and silicone oils.

9. Process according to claim 7 or 8, **characterized in that** the compound which is insoluble in the reaction medium is added at the beginning of the reaction.

10. Process according to claim 7 or 8, **characterized in that** the compound which is insoluble in the reaction medium is added before the stage of reduction of the palladium.

11. Process according to one of the preceding claims, **characterized in that** the compounds comprising a conjugated ethylenic unsaturation are chosen from the group consisting of diolefins, allyl alcohols, allyl ethers, allyl esters and allyl halides.

12. Process according to claim 11, **characterized in that** the diolefin is butadiene.

13. Process according to one of claims 7 to 12, **characterized in that** the palladium deposited on the insoluble compound is recovered by treatment of the said insoluble compound after separation from the reaction medium.

14. Process according to claim 13, **characterized in that** the treatment of the insoluble compound comprising the reduced palladium is a dissolution of the palladium by attack by a strong acid.

## Patentansprüche

1. Verfahren zur Herstellung von ungesättigten oder gesättigten Carbonsäuren aus Verbindungen, umfassend eine ethylenisch oder acetylenisch ungesättigte Bindung, die mit einer weiteren ungesättigten Bindung oder einer Elektronendonorgruppe konjugiert ist, durch Reaktion von Kohlenmonoxid und Wasser in Gegenwart eines Katalysators auf der Basis von Palladium, **dadurch gekennzeichnet, dass** es darin besteht, das Reaktionsmedium nach dem Ende der Hydroxycarbonylierungsreaktion durch Extraktion des Kohlenmonoxids aus besagtem Reaktionsmedium durch Behandlung mit einem Gas, um eine Konzentration an CO kleiner als 600 ml CO pro Liter Lösung zu erhalten, dann Behandlung des Reaktionsmediums mit Wasserstoff, um das Palladium auf die Oxidationsstufe Null zu reduzieren, zu behandeln und das ausgefällte Palladium vom Reaktionsmedium abzutrennen.

2. Verfahren gemäß Anspruch 1, **dadurch gekennzeichnet, dass** die Konzentration an Kohlenmonoxid in dem Reaktionsmedium vor dem Schritt zur Behandlung mit Wasserstoff kleiner als 10 mmol/l ist.

3. Verfahren gemäß Anspruch 2, **dadurch gekennzeichnet, dass** die Konzentration an Kohlenmonoxid in dem Reaktionsmedium vor dem Schritt zur Behandlung mit Wasserstoff zwischen 0,001 ml/l und 600 ml/l liegt.

4. Verfahren gemäß einem der Ansprüche 1 bis 3, **dadurch gekennzeichnet, dass** das Gas zum Austreiben des Kohlenmonoxids ausgewählt ist aus der Gruppe, die Stickstoff, die Edelgase oder Wasserstoff umfasst.

5. Verfahren gemäß einem der Ansprüche 1 bis 4, **dadurch gekennzeichnet, dass** der Schritt zur Reduktion des Palladiums auf die Oxidationsstufe Null unter einem Wasserstoffdruck zwischen 1 bar und 100 bar durchgeführt wird.

6. Verfahren gemäß einem der Ansprüche 1 bis 5, **dadurch gekennzeichnet, dass** der Schritt zur Reduktion des Palladiums auf die Oxidationsstufe Null bei einer Temperatur zwischen 20 °C und 150 °C durchgeführt wird.

7. Verfahren gemäß einem der vorhergehenden Ansprüche, **dadurch gekennzeichnet, dass** eine in dem Reaktionsmedium unlösliche Verbindung zu besagtem Medium hinzugefügt wird, dann nach dem Schritt zur Reduktion des Palladiums auf die Oxidationsstufe Null von besagtem Medium abgetrennt wird.

8. Verfahren gemäß Anspruch 7, **dadurch gekennzeichnet, dass** die in dem Reaktionsmedium unlösliche Verbindung ausgewählt ist aus der Gruppe, die die Aktivkohlen, Aluminiumoxid, Siliciumdioxid, Zirkoniumoxid, Ceriumoxid, die Polystyrenschäume, die Siliconöle umfasst.

9. Verfahren gemäß Anspruch 7 oder 8, **dadurch gekennzeichnet, dass** die in dem Reaktionsmedium unlösliche Verbindung am Reaktionsanfang hinzugefügt wird.

10. Verfahren gemäß Anspruch 7 oder 8, **dadurch gekennzeichnet, dass** die in dem Reaktionsmedium unlösliche Verbindung vor dem Schritt zur Reduktion des Palladiums hinzugefügt wird.

11. Verfahren gemäß einem der vorhergehenden Ansprüche, **dadurch gekennzeichnet, dass** die Verbindungen, die eine konjugierte ethylenisch ungesättigte Bindung umfassen, ausgewählt sind aus der Gruppe, die die Diolefine, die Allylalkohole, Allylether, Allylester und Allylhalogenide umfasst.

12. Verfahren gemäß Anspruch 11, **dadurch gekennzeichnet, dass** das Diolefin Butadien ist.

13. Verfahren gemäß einem der Ansprüche 7 bis 12, **dadurch gekennzeichnet, dass** das auf der unlöslichen Verbindung abgeschiedene Palladium durch Behandlung besagter unlöslicher Verbindung nach der Abtrennung vom Reaktionsmedium zurückgewonnen wird.

14. Verfahren gemäß Anspruch 13, **dadurch gekennzeichnet, dass** die Behandlung der unlöslichen Verbindung, die das reduzierte Palladium umfasst, ein Auflösen des Palladiums durch Angriff mit einer starken Säure ist.
